# EUROPEAN PATENT SPECIFICATION

(11) **EP 1 830 189 B1**
(45) Date of publication and mention of the grant of the patent: **05.09.2012**
(21) Application number: 05819685.8
(22) Date of filing: 22.12.2005
(51) Int. Cl.: G01N 33/543, G01N 33/574

(54) **REAGENT AND METHOD FOR ASSAYING PROSTATE SPECIFIC ANTIGEN**
Reagens und Verfahren zum Nachweis von Prostata-spezifischem Antigen
Réactif et procédé de détection de l'antigène prostatique spécifique

(30) Priority: 24.12.2004 JP 2004373382
(43) Date of publication of application: 05.09.2007
(73) Proprietor: Sekisui Medical Co., Ltd., Tokyo 103-0027 (JP)
(72) Inventor: HONJO, Yuki, Daiichi Pure Chemicals Co. Ltd., Ryugasaki-shi, Ibaraki 3010852 (JP); AKIMOTO, Yuka, Daiichi Pure Chemicals Co. Ltd., Ryugasaki-shi, Ibaraki 301-0852 (JP); KOTANI, Kazuo, Daiichi Pure Chemicals Co., Ltd., Tokyo 1030027 (JP); YAGO, Hirokazu, Daiichi Pure Chemicals Co. Ltd., Ryugasaki-shi, Ibaraki 3010852 (JP); MATSUO, M., Daiichi Pure Chemicals Co., Ltd., Tokai-mura, Naka-gun, Ibaraki 3191182 (JP); MIYAZAKI, O., Daiichi Pure Chemicals Co., Ltd., Tokai-mura, Naka-gun, Ibaraki 3191182 (JP); SAITO, Kazunori, Daiichi Pure Chemicals Co., Ltd., Ryugasaki-shi, Ibaraki 3010852 (JP)
(74) Representative: Forstmeyer, Dietmar
(86) International application number: PCT/JP2005/023568
(87) International publication number: WO 2006/068206

(56) References cited:
- JP-A- 2000 002 704
- JP-A- 2001 108 681
- JP-A- 2001 108 681
- JP-A- 2001 289 853
- JP-A- 2001 311 733
- JP-A- 2005 106 609
- JP-A- 2005 326 150
- US-A1- 2005 069 967
- ZHOU Z ET AL: "Technicon Immuno 1 PSA assay measures both free and alpha-1-antichymotrypsin-complexed prostate-specific antigen on an equimolar basis." JOURNAL OF CLINICAL LABORATORY ANALYSIS 1996, vol. 10, no. 3, 1996, pages 155-159, XP002477142 ISSN: 0887-8013
- ZHOU Z ET AL: "Equivalent recognition of free and ACT-complexed PSA in a monoclonal-polyclonal sandwich assay is conferred by binding specificity of the monoclonal antibody." JOURNAL OF CLINICAL LABORATORY ANALYSIS 1998, vol. 12, no. 4, 1998, pages 242-249, XP002477143 ISSN: 0887-8013

## Description

### Technical Field

The present invention relates to a reagent for assaying PSA and a method of assaying PSA by an immunological agglutination reaction method. More particularly, the present invention relates to a reagent for assaying PSA and a method of assaying PSA by measuring PSA by latex-enhanced turbidimetric immunoassay (LTIA) with adjusting reactivity to free PSA (fPSA) and complex-type PSA (PSA-ACT).

### Background Art

Prostate cancer is a malignant disease found in males that is drastically increasing in particular in U.S.A. and Japan. Since prostate cancer is characterized in that it is a tumor that grows slowly and that radiation therapy and antiandrogenic therapy tend to be effective to prostate cancer, early detection is a key point.

Here, prostate specific antigen (hereinafter, simply "PSA") secreted from prostate epithelial cells and also produced by prostate cancer cells is a kind of serine protease, which is a glycoprotein having a molecular weight of 33,000 to 34,000 daltons. PSA includes a complex-type one that bound to a protease inhibitor in the blood and a nonbinding, free-type prostate specific antigen (hereinafter, sometimes "fPSA"). Most of PSA in blood is of a complex-type, including an α1-antichymotrypsin complex prostate specific antigen (hereinafter, sometimes "PSA-ACT") and an α2-macroglobulin complex prostate specific antigen. Among these, only two, i.e., fPSA and PSA-ACT, can be measured by immunoassay. More than 20 types of kits for assaying a total amount of serum PSA that are practically applied in Japan have encountered a problem that the kits differ in degree of deviation between measured values and true values depending on suspected fluids. This would be mainly because on a kit by kit basis, each kit has a different reactivity of the measuring reagent with fPSA and PSA-ACT.

That is, there has been a problem that in a so-called immunological agglutination reaction assay by an method reaction using a latex or the like from among immunological methods, the antigen-antibody reaction is performed in one step, so that the reactivity of a monoclonal antibody to PSA differs for different PSA, i.e., the reactivity of the monoclonal antibody with fPSA and the reactivity of the monoclonal antibody with PSA-ACT differ from one another, thus causing errors in measured results.

As a conventional technique for solving the problem, for example, Patent Document 1 describes that binding an antibody to a region capable of binding α1-antichymotrypsin in fPSA to bring the molecular weight of the bound fPSA and that of PSA-ACT closer to each other and making the number of epitopes uniform between bound fPSA and PSA-ACT, thus bringing the reactivity of bound fPSA and the reactivity of PSA-ACT with an antibody to PSA closer to each other enable measurement of a carrier sensitized with the antibody and the both types of PSAs in an equimolar reactivity (hereinafter called, "equimolarity").
Patent Document 1: JP 2001-311733 A

JP 2001 108681 A discloses a reagent capable of obtaining the correct blood concentration of a substance to be determined, e. g. PSA, regardless of the present form of the substance to be determined, a reagent for specifically determining only a complex, and a new method for measuring both.

### Disclosure of the Invention

### Problem to be solved by the Invention

However, the above-mentioned conventional assay has a problem that it is necessary to provide a special antibody that reacts with fPSA alone in advance. Moreover, a two-step reaction has been required, that is, first a pretreatment is performed in which the special antibody is reacted with fPSA and then carriers sensitized with an antibody which reacts with both PSAs, respectively were used for the measurement of total PSA.
Therefore, development of a method for measuring total PSA more easily and more accurately has been keenly desired. Note that unless otherwise indicated specifically, measurement of PSA means measurement of total amount of PSA.

### Means for Solving the Problems

The inventors of the present invention have made extensive studies on a method of assaying PSA by an immunological agglutination method and found that in a reagent for assaying PSA constituted by at least three antibodies having different recognition sites for free PSA contained and PSA-ACT, at least two of the antibodies being sensitized to a carrier and capable of reacting with the both antigens, adjusting a mixing ratio of each antibody enables adjustment of reactivities of the both antigens, thus accomplishing the present invention.
That is, the present invention has the following structures:
(1) a reagent for assaying an antigen by an immunological agglutination reaction, including:
   at least three types of antibodies capable of reacting with both PSA-ACT and free PSA and having different recognition sites therefor, in which:
   at least two types of antibodies out of the at least three types of antibodies are each sensitized to a carrier and react with both antigens; and
   mixing ratios of the antibodies are adjusted so that both antigens can be determined in (or with) a predetermined molar reactivity when the antibodies each react with PSA-ACT and fPSA.
(2) the reagent for assaying an antigen according to item (1), in which the predetermined reactivities are equimolarities;
(3) the reagent for assaying an antigen according to the above-mentioned item (2), in which the reagent includes three types of antibodies;
(4) the reagent for assaying an antigen according to any one of the above-mentioned items (1) to (3), in which all the antibodies are sensitized to the respective carriers;
(5) the reagent for assaying an antigen according to any one of the above-mentioned items (1) to (4), in which the carrier sensitized with one of the antibodies and the carrier sensitized with another of the antibodies have different particle sizes from each other;
(6) the reagent for assaying an antigen according to any one of the above-mentioned items (1) to (5), in which the antibodies are monoclonal antibodies;
(7) a reagent for assaying PSA by an immunological agglutination reaction, including:
   latexes sensitized with three types of monoclonal antibodies capable of reacting with both PSA-ACT and fPSA and having different recognition sites therefor, in which
   latexes sensitized with the two types of antibodies out of the three types of antibodies have a particle size larger than that of the latex sensitized with the rest-type of antibody, and
   mixing ratios of the antibodies are adjusted so that the antibodies each exhibit predetermined molar reactivities with the both antigens;
(8) the reagent for assaying PSA according to above-mentioned item (7), in which the carriers sensitized with the respective antibodies are included in a single reagent;
(9) the reagent for assaying PSA according to above-mentioned item (7) or (8), in which the latex sensitized with the rest-type of antibody has a particle size of 0.02 µm to 0.22 µm;
(10) a method of producing a PSA assaying reagent by an immunological agglutination reaction, including:
   providing three carriers sensitized with respective antibodies capable of reacting with both PSA-ACT and fPSA and having different recognition sites therefor;
   setting in advance a mixing ratio of two different antibody-sensitized carriers out of the three types of antibody-sensitized carriers to a predetermined range; and
   then, adding the rest antibody-sensitized carrier to a mixture of the two types of antibody-sensitized carriers to adjust the both antigens and the two types of antibodies to exhibit predetermined molar reactivities;
(11) the reagent for assaying an antigen according to above-mentioned item (10), in which the predetermined reactivities are equimolarities.
(12) a method of assaying PSA in an analyte by an immunological agglutination reaction, including:
   assaying PSA in an analyte using a reagent for assaying PSA, in which:
      the reagent for assaying PSA includes carriers sensitized with three types of antibodies capable of reacting with both PSA-ACT and fPSA having different recognition sites therefor;
      carriers sensitized with two types of antibodies out of the three types of antibodies have a larger particle size than that of a carrier sensitized with the rest-type of antibody; and
      a mixing ratio of each of the antibodies is adjusted so that the both antigens and the antibodies exhibit predetermined molar reactivities; and
(13) the method of assaying PSA in an analyte according to above-mentioned item (12), in which the predetermined molar reactivities are equimolarities.

### Effects of the Invention

The reagent for assaying PSA according to the present invention can be prepared simply and at low cost by appropriately combining antibodies obtained by a conventional method without using antibodies having special reactivity. When all the antibodies are sensitized to a carrier, the reagent has excellent stability and quality control of the reagent is simple, so that stable supply of the reagent can be expected. In this case, reagents including sensitized antibodies can be used as a single reagent, and measurement can be performed in a one-step reaction. This makes measurement simple and shortens measuring time.

### Brief Description of the Drawings

[Fig. 1] Fig. 1 is a photograph illustrating reactivity of each antibody to fPSA and PSA-ACT (Western blotting).
[Fig. 2-1] Fig. 2-1 is a graph showing the effect of addition of # 16Lx on c/f ratio (test example 1-1).
[Fig. 2-2] Fig. 2-2 is a graph showing the effect of addition of # 16Lx on c/f ratio (test example 1-2).
[Fig. 3] Fig. 3 is a graph showing the effect of addition of free # 16 antibody on c/f ratio.
[Fig. 4] Fig. 4 is a graph showing the effect of improvement of c/f ratio depending on latex size of # 16Lx.

### Best Mode for carrying out the Invention

### (Target sample)

A target sample of the reagent for assaying PSA according to the present invention is a sample that contains PSA. Examples of such include blood, serum, plasma, lymphocyte culture suspension, urine, spinal fluid, saliva, sweat, ascitic fluid, and extracts from cells and organs. When the antigen is PSA, any biological sample that may contain PSA-ACT complex is a target.

### (Antigen)

The antigen which is a target of the measurement is any antigen that can be assayed by an immunological agglutination method. For example, among analytes which serve as target samples, those antigens having both existence forms, i.e., a free-type and a complex-type that forms a complex with a coexistent substance are targeted. The coexistent substance includes protease inhibitors that form a complex with a free-type antigen to inhibit the activity of the free-type antigen, transporter for free-type antigen, and proteins such as albumin. More specifically, examples of free-type antigens include protease (PSA, MMP (Matrix Metalloproteinase), trypsin, chymotrypsin, elastase, cathepsin), hormones (T3 (Triiodothyronine) and T4 (thyroxine), as well as drugs. For example, PSA includes fPSA and PSA-ACT; T3 and T4 include a complex-type that is bound to thyroxine-bound globulin (transporter protein) and a free-type; and drugs in blood include a complex-type that is bound to albumin and a free-type.

### (Antibody)

The antibodies used in the present invention include at least two types that react with both free-type and complex-type antigens as a target of assay. The antibody may be polyclonal antibodies obtained by immunizing rabbit, sheep, goat or the like or monoclonal antibodies. Of these, monoclonal antibodies are particularly preferable. While the whole antibody may be used, fragments obtained by enzymatically cleaving the whole antibody by a conventional method may also be used. The monoclonal antibody to be used may be obtained by a conventional method.
In the present invention, when a monoclonal antibody is used, an antibody suited for the combination can be appropriately selected by, for example, a sandwich method or a competition method.
The antibodies used in the present invention must have different reactivity with the antigen from one another. Here "different reactivity" means a difference in affinity, binding power, epitope or the like of the antibody for the antigen. In this case, "difference in epitope" also includes the case where recognition sites overlap partly or located closely.

### (Carrier)

The carrier used in the present invention for sensitization with the antibody is not particularly limited but a latex is preferable. The latex used may be a latex made of polystyrene commonly used in a physical adsorption method used in immunoassays and the material of the latex is not particularly limited as far as the latex is suited for the sensitization method. In addition, particles of various materials such as styrene-butadiene copolymers, (meth) acrylic acid ester polymer, latex, gelatin, liposome, microcapsules, silica, alumina, carbon black, metal compounds, metals, ceramics, and magnetic substances may be used. The method of sensitizing the latex with an antibody may be a physical adsorption method commonly used. However, a chemical bonding method may also be used.
The latexes used in the present invention may be of the same type or two or more different types. It is preferable that two or more types of latexes with different particle diameters be used. Further, a mixture of latex particles with different particle sizes may be used.

### (Selection of antibody)

Antibodies can be selected as follows. Description is made of an example in which the antigen is PSA and three antibodies are selected. It is preferable to select an antibody having high reactivity or high titer with PSA-ACT and fPSA by an ELISA method or the like, and it is also preferable to select a carrier capable of providing a high sensitivity for assay. Then, a combination of antibodies are selected, which provides little difference in reactivity with PSA-ACT and fPSA and also provides high sensitivity for assays in the case where two types of antibodies are selected from the selected high titer antibodies and are sensitized to the carrier. Such antibodies are used as main reaction antibodies.
Then, another antibody is selected such that the other antibody can adjust reactivity of the main reaction antibody with PSA-ACT or fPSA when the other antibody is allowed to coexist in the main reaction. The selected antibody is used as an antibody for adjusting reaction (hereinafter called, "reaction adjusting antibody"). Note that the reaction adjusting antibody may be sensitized to a carrier. The carrier to which the reaction adjusting antibody is sensitized preferably has a size smaller than that of a carrier to which the main reaction antibody is sensitized. The reaction adjusting antibody preferably has different reactivity with PSA-ACT or fPSA.

### (Mixing ratio of antibodies)

The mixing ratio of antibodies has to be adjusted so that a free-type antigen and a complex-type antigen can be determined in (or with) a predetermined molar reactivity The present invention is characterized in that the molar reactivity of antigen can be adjusted by adjusting the mixing ratio of antibodies and the molar reactivity of antigen can be adjusted in a predetermined range depending on the purpose and required precision of assay. In particular, concentration of the free-type antigen and complex-type antigen can be measured at the same reactivity, when the mixing ratio of antibodies are adjusted so that both antigens show equimolarity
Here, the equimolarity means that reactivities of agglutination of carriers sensitized with antibodies when the antibodies are reacted with antigens are substantially the same between a free-type antigen and a complex-type antigen. Specifically, when the antigen is PSA and the PSA includes fPSA and PSA-ACT at the same concentration, then the concentrations of PSAs assayed by agglutination reaction are substantially the same. Further, the "substantially the same" means that the ratio of the agglutination signals of one antigen to the agglutination signal of the other antigen is 80% to 120%, preferably 90% to 110%.

According to the present invention, it has been found that appropriate adjustment of mixing ratio of antibodies in immunological agglutination reaction of antigens can result in antigen-antibody reactions at predetermined molar reactivities. For example, when three types of antibodies that react with both a PSA-ACT and an fPSA and have different recognition sites are sensitized to respective carriers to prepare reagents having equimolarity, the mixing ratio of each antibody is adjusted as follows. First, using a carrier sensitized with each of two types of antibodies, a ratio of agglutination signal of one antigen to that of the other antigen per mol (PSA-ACT/fPSA ratio, hereinafter, sometimes referred to simply c/f ratio) is obtained, and then a carrier sensitized with another antibody is added such that the c/f ratio is 80% to 120%, preferably 90% to 110%.

For the adjustment of c/f ratio, free antibodies not sensitized to carriers as well as antibodies sensitized to carries may be used. The antibody for the adjustment of c/f ratio (reaction adjusting antibody) may be of one type or two or more than two types. Note that when a free antibody is used, a separate reagent containing the free antibody has to be prepared, so that the free antibody will not react with a carrier sensitized with other antibody by agglutination reaction.

When a reaction adjusting antibody is sensitized to a carrier, it is preferable to use a carrier having a particle diameter smaller than that of the carrier to which a main reaction antibody is sensitized. The use of the carrier facilitates the adjustment of c/f ratio. Specifically, the reaction adjusting antibody has a particle diameter of in the range of preferably 0.02 µm to 0.22 µm, more preferably 0.05 µm to 0.09 µm. The particle size of a carrier to which main reaction antibody is sensitized is preferably 0.1 µm to 0.5 µm. More preferably, the particle size of the carrier to which the reaction adjusting antibody is sensitized is 2/3 to 1/10, still more preferably 1/5 to 1/8, of the particle size of the carrier to which the main reaction antibody is sensitized.
Here, the "mixing ratio of an antibody" means concentration ratio of antibodies. Accordingly, when the antibodies are respectively sensitized to carriers in a certain ratio, mixing ratio of the antibodies corresponds to concentration ratio of the carriers.

### (Buffer solution)

In the present invention, the antigen-antibody reaction is performed in a buffer solution. Type, concentration, and pH of the buffer solution are selected so that the antigen-antibody reaction is performed optimally. For example, phosphate buffer solutions, Tris-HCl buffer solutions, carbonate buffer solutions, glycine buffer solutions, and a Good's buffer solution can be used. The concentration of the buffer solution during assay is about 10 mM to about 500 mM. The pH of the buffer solution is mostly from a neutral region to basic region, and falls usually in the range of 7.0 to 9.5.

### (Method of assaying agglutination signal)

Assay of agglutination signal may be performed by any assaying method that is usually used for assaying immunological agglutination reaction and includes assays of absorbance, particle number, and particle size as well as measurement of scattered light.
For example, a method of assaying PSA is performed as follows. An assaying reagent contains at least three types of antibodies, two or more of which antibodies are antibodies that are bound to a carrier made of latex or the like and react with both fPSA and PSA-ACT. Therefore, when such an antibody and an antigen are reacted, fPSA and PSA-ACT react with the antibodies sensitized to the carrier to agglutinate. This is because in the case of the agglutination reaction using an antibody sensitized to a carrier, if one antigen molecule binds to two or more antibodies sensitized to respective carriers, this can be theoretically observed as agglutination. Accordingly, measurement of degree of the agglutination and comparison with the degree of agglutination of a standard solution of which the PSA concentration is known enables quantitation of total PSA concentration in a sample.

While detection of an agglutination signal may be performed by various methods, it is convenient to use a general-purpose biochemical analyzer. For example, detection of an agglutination signal can be performed by adding a reagent to a sample containing an antigen, leaving the mixture to stand at a predetermined temperature and for a predetermined time, measuring absorbance during the time, detecting an amount of change in absorbance, and calculating the antigen concentration of a test body fluid from a calibration curve of the standard solution. In the case of a latex agglutination method, usually absorbance at a wavelength of 500 nm to 900 nm is used and generally an amount of change in absorbance of the reaction is used for the quantitation.

### (Method of producing reagent)

The PSA assay reagent of the present invention is produced as follows.
Three carriers sensitized with respective antibodies that react with both PSA-ACT and fPSA and have different recognition sites thereof are provided, the mixing ratio of a two different antibody-sensitized carriers out of the three types of antibody-sensitized carriers is set to a predetermined range, and the rest antibody-sensitized carriers is added to an above-mentioned mixture containing the two types of antibody-sensitized carriers thereby performing adjustment so that the antigen and the antibodies exhibit predetermined molar reactivity.

### Example

### [Preparation of monoclonal antibody]

### 1. Immunization

### (1) Immunogen

As an immunogen, human sperm-derived purified PSA (manufactured by SCIPAC Corporation, Code No. P 117-7, degree of purification: 96%) was used. The purified PSA was used after dialysis with PBS.

### (2) Immunization method

The PSA solution and CFA (complete Freund's adjuvant: manufactured by GIBCO) were mixed in a ratio of 1:1 and emulsified. The emulsion was subcutaneously administered to 6-week female Balb/C mice on the back in an amount of 25 µg PSA/animal. Additional immunization was performed three times every two weeks and furtherly a PSA solution (25 µg/animal) was intraperitoneally administered to the mice three days before cell fusion.

### 2. Cell fusion

The spleen of the mice after immunization of the PSA was extirpated and spleen cells were taken out. The spleen cells and mouse myeloma cells SP2/O-Ag14 were mixed in a ratio of 6:1 and cell fusion was allowed to take place in the presence of 50% polyethylene glycol 1540 (Wako Pure Chemical Industries Co., Ltd.). The fused cells were suspended in HAT medium to a density of 2.5x 10⁶ as spleen cell and then dispensed to a 96-well cultivation plate (manufactured by CORNING CO.) in an amount of 0.2 ml/well. The plate was incubated at 37°C for 2 weeks in a 5% CO₂ incubator.

### 3. Screening

Using a supernatant in each culture dish dispensed with fused cells, a well that reacts with both fPSA and PSA-ACT was selected by the following ELISA method.
(1) Material: antigen
   1) PSA (PROSTATE SPECIFIC ANTIGEN): SCIPAC, Code No. P117-7
   2) PSA-ACT Complex (PSA-α1-ANTICHYMOTRYPSIN): SCIPAC, Code No. P192-3
(2) Method
   1) 5 µg/ml of goat anti-mouse IgG(Fc) antibody (manufactured by Jackson Co.) was coated (50 µl/well) on a plate for ELISA (Nunc) and left to stand at 4°C overnight.
   2) The plate was washed with a washing solution (0.05% Tween 20-PBS) three times (400 µl/well), and then a blocking solution (0.05% Tween 20-PBS) was dispensed to each well to make 200 µl and left to stand at room temperature for 1 hour.
   3) After the blocking solution was removed, a culture supernatant of each culture dish in the plate dispensed with fused cells was dispensed to each 50 µl/well in ELISA plate, and then left to stand at room temperature for 1 hour.
   4) The plate was washed three times with a washing solution (0.05% Tween 20-PBS) , and then 50 µl of fPSA or PSA-ACT diluted with 0.05% Tween 20-PBS to 5 ng/ml was dispensed to each well, which was then left to stand at room temperature for 1 hour.
   5) After three times washing the plate with a washing solution 50 µl/well of an HRP-rabbit anti-human PSA antibody (x500) was dispensed to each well and left to stand at room temperature for 1 hour. Note that the HRP-rabbit anti-human PSA antibody was prepared by using a rabbit anti-human PSA antibody (manufactured by DAKO CO.) and peroxidase (manufactured by Toyobo Co., Ltd.) according to a periodate method.
   6) After washing the plate three times, 50 µl/well of an OPD color solution was dispensed to each well and left to stand at room temperature for 10 minutes.
   7) 50 µl/well of a stopping solution (1.5-N sulfuric acid) was added to each well to stop the reaction and then absorbance at a wavelength of 492 nm was measured using a plate reader.

### 4. Cloning

By the above-mentioned screening, a cell line in the well that reacted with both fPSA and PSA-ACT was cloned by limiting dilution method to establish a hybridoma. As a result, 20 types of established hybridomas were obtained.

### 5. Preparation of monoclonal antibody

To 8-week female Balb/C mice to which 0.5 ml of pristane was intraperitoneally injected 2 weeks in advance, 0.5×10⁶ cells/animal of each hybridoma was intraperitoneally administered. After two weeks, ascitic fluid was collected and IgG fraction was purified therefrom by Protein A Column (manufactured by Amersham Co.). As a result, purified fractions of 20 types of monoclonal antibodies were obtained.

### [Selection of monoclonal antibody and carrier]

The 20 types of monoclonal antibodies obtained as described above were sensitized to latex, and latex agglutination was studied as described below. As a result, three types of antibodies having large signals, i.e., antibody # 14MoAb, # 16MoAb, and # 17 MoAb (respective definitions are described hereinafter) as well as combinations thereof with latex were obtained.
1. First selection of antibody
   (1) 0.312 µl of 1% latex solution diluted with 20 mM Tris HCl (pH 8.5) and each of 20 types of MoAb solutions (0.6 Abs) were mixed in a ratio of 1:1 (vol/vol) and stirred at 4°C for 2 hours.
   (2) 2 volumes of 0.4% BSA in 20 mM Tris HCl (pH 8.5) was added to the mixture and the resultant was stirred at 4°C for 1 hour.
   (3) After centrifugation, the supernatant was removed and the residue was suspended in 5 mM MOPS (pH 7.0) and adjusted to 2 Abs at 600 nm of Abs.
   (4) Two out of 20 types of MoAb-Lx (antibody-sensitized latex) were mixed in a ratio of 1:1 (vol/vol) to prepare reagent 2 (all combinations were prepared as described above and studied in the same manner as follows).
   (5) Using reagent 1 (30 mM HEPES, pH 7.0, 0.1% BSA, 0.5 M NaCl) and reagent 2, 26 ng/ml fPSA and PSA-ACT were measured on Hitachi 7170 type Automatic Analyzer.
   (6) 12 types of combinations of MoAb's that agglutinate (having high absorbance) and 8 types of MoAb were selected.
2. Second selection of antibody
   (7) 2.9, 8.7 or 26 ng/ml of fPSA or PSA-ACT were measured by Hitachi 7170 type Automatic Analyzer and combinations of antibodies that provide high absorbances and PSA-ACT/fPSA ratio near 1 at each concentration were selected as main reaction antibodies (#14MoAb, # 17MoAb).

   Note that a monoclonal antibody that exhibits a difference in reactivity between antibodies when reacted with each of the second selected combination of antibodies was selected as a reaction adjusting antibody (# 16MoAb).
3. Selection of latex
   (8) Types of latex: 0.39, 0.31,0.30, or 0.23 µm (for high reactivity latexes: using latexes providing high absorbance), or 0.22, 0.087, or 0.055 µm (for reaction adjusting latexes) latexes, antibody-sensitized latexes were prepared according to (1) to (4) above.
   (9) 2.9, 8.7 or 26 ng/ml fPSA or PSA-ACT were measured by Hitachi 7170 type Automatic Analyzer and combinations that provided high absorbance and PSA-ACT/fPSA ratio near 1 at each concentration were selected.
   (10) Combinations of # 14MoAb-0.229 µm, # 17MoAb-0.385 µm, and # 16MoAb-0.055 µm were selected.
   (11) For each combination, optimal conditions of MoAb concentration, latex concentration, antibody-sensitized latex concentration, sensitization conditions (buffer solution, pH, and time), and mixing ratio of antibody-sensitized latexes were set, and the following experimental examples were carried out.

### [Analysis of each monoclonal antibody by Western blotting method]

The reactivities of the three types of antibodies obtained as described above (# 14MoAb, # 16MoAb, and # 17MoAb) with fPSA and PSA-ACT were analyzed by the following Western blotting method.

### 1. Material

(1) Antigen
   1) PSA (PROSTATE SPECIFIC ANTIGEN): SCIPAC, Code No. P117-7
   2) PSA-ACT Complex (PSA-α1-ANTICHYMOTRYPSIN): SCIPAC, Code No. P192-3
(2) Antibody
   1) Anti-PSA MoAb (63214 is abbreviated as # 14): antibody obtained by the above-mentioned "preparation of monoclonal antibody"
   2) Anti-PSA MoAb (63216 is abbreviated as # 16): same as above
   3) Anti-PSA MoAb (63217 is abbreviated as # 17): same as above
   4) Anti-PSA MoAb (No. 56): manufactured by Japan Clinical Laboratories, Inc, lot. 031-02-003
   5) Anti-PSA MoAb (4D10): manufactured by Japan Clinical Laboratories, Inc, lot. 031-01-004
   6) Normal mouse IgG: (own preparation)
   7) Rabbit Anti-Human PSA (Rb anti-PSA PoAb): manufactured by DAKO, Code No. A0562
   8) Rabbit Anti-Human ACT (Rb anti-PSA PoAb): manufactured by DAKO, Code No. A0022
(3) Labeled antibody
   1) Goat Anti-Rabbit Ig's HRP Conjugate: BioSource, ALI3404, lot. 5701
   2) Goat (F (ab'2)) Anti-Mouse Ig's HRP Conjugate: BioSource, AMI4404
(4) Others
   1) Blocking solution, diluent: 1% BSA-PBS-Tween (0.05%-Tween 20), washing solution: PBS-Tween (0.05%-Tween 20)
   2) OPD color solution, stop solution (ELISA): common procedure
   3) DAB staining solution (Western Blotting): common procedure

### 2. Method

1) Each antigen (fPSA or PSA-ACT) was mixed with an SDS treatment solution (2-mercaptoethanol-free) and boiled for 5 minutes.
2) The resultant was applied to PAG Mini 10/20 (2 µg/lane) and SDS-PAGE was performed (40 mA, 1 hour).
3) After the electrophoresis, proteins were transferred from the gel to a PVDF membrane using a semi-dry blotter according to a conventional method.
4) The membrane after the transfer was blocked with 5% Skim Milk-PBS-Tween (4°C/overnight).
5) Each MoAb diluted to a concentration of 5 µg/ml was reacted at room temperature for 2 hours.
6) After washing three times, Goat (F(ab'2) Anti-Mouse Ig's HRP Conjugate (diluted x5000) was reacted at room temperature for 1 hour.
7) After washing the membrane three times, the resultant was dyed with DAB according to a conventional method.
8) At the time when an appropriate dyed image was obtained, the membrane was washed and dried.

### 3. Results

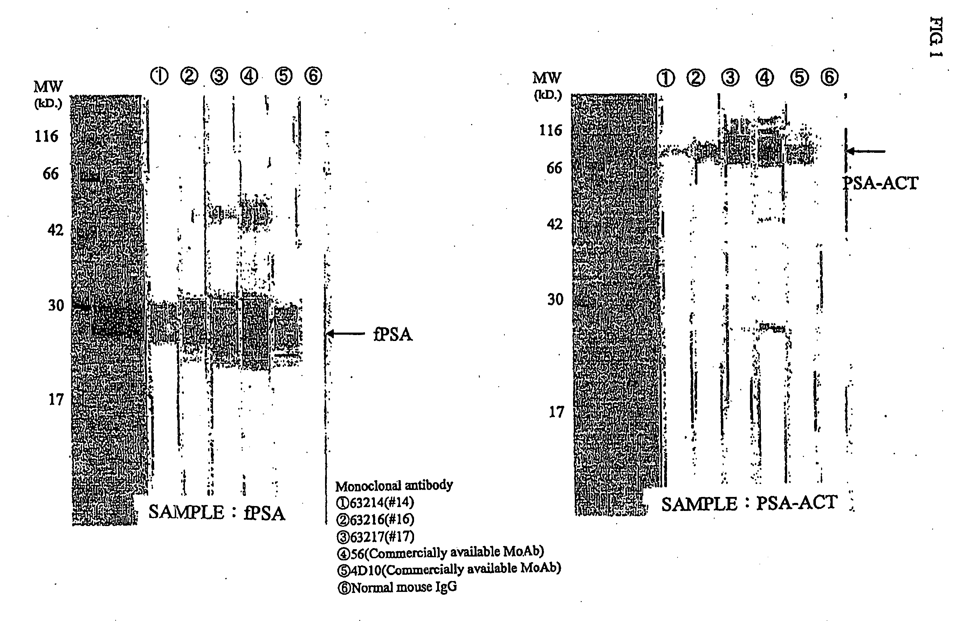

Fig. 1 shows a dyed image obtained by the Western blotting method. Fig. 1 indicates that the monoclonal antibodies # 14, # 16, and # 17 of the present invention each react with both fPSA and PSA-ACT.

### Method of preparing antibody-sensitized latex

The three types of monoclonal antibodies (# 14, # 16, and # 17) obtained as described above were sensitized to respective carriers using the following material and method.

### 1. Material

(1) anti-PSAmonoclonal antibody
   63214 (# 14)
   63217 (# 17)
   63216 (# 16)
   (all the above-mentioned monoclonal antibodies are in PBS solution)
(2) Latex
   Latex for # 14: polystyrene latex having an average particle diameter of 0.23 µm (manufactured by SEKISUI CHEMICAL CO., LTD.)
   Latex for # 17: polystyrene latex having an average particle diameter of 0.39 µm (manufactured by SEKISUI CHEMICAL CO., LTD.)
   Latex for # 16: polystyrene latex having an average particle diameter of 0.05 µm (manufactured by SEKISUI CHEMICAL CO., LTD.)

### 2. Method of preparing antibody-latex complex-containing solution

(1) Method of preparing a # 14 antibody-latex complex (# 14Lx)-containing solution
   1) A latex and an antibody were each diluted with 20 mM glycine at pH 9 to prepare a 1% latex solution and a 0.4 mg/ml # 14 antibody solution. These solutions were mixed in a ratio of 1:1 (vol/vol) and stirred for about 1 hour.
   2) 0.1 volume of a blocking solution (10% BSA) was added to 2 volumes of the resultant mixed solution and the obtained mixture was stirred for about 1 hour.
   3) After dialysis against 5 mM MOPS (pH 7.0) solution, the dialyzed mixture was diluted to 3 Abs/ml (600 nm) to obtain a # 14 antibody-latex complex (# 14Lx)-containing solution.
(2) Method of preparing a # 17 antibody-latex complex (# 17Lx)-containing solution
   1) A latex and an antibody were each diluted with 20 mM Tris pH 8.0 to prepare a 0.3% latex solution and a 0.1 mg/ml # 17 antibody solution. These solutions were mixed in a ratio of 1:1 (1 volume + 1 volume) and stirred for about 1 hour.
   2) 0.03 volume of a blocking solution (10% BSA) was added to 2 volumes of the resultant mixed solution and the obtained mixture was stirred for about 1 hour.
   3) After dialysis against 5 mM MOPS (pH 7.0) solution, the dialyzed mixture was diluted to 2.7 Abs/ml (600 nm) to obtain a # 17 antibody-latex complex (# 17Lx)-containing solution.
(3) Method of preparing a # 16 antibody-latex complex (# 16Lx)-containing solution
   1) A latex and an antibody were each diluted with 20 mM Tris pH 8.5 to prepare a 5% latex solutions and a 0.6 mg/ml # 16 antibody solution. These solutions were mixed in a ratio of 1:1 (1 vol + 1 vol) and stirred for about 2 hour.
   2) 0.4 volume of a blocking solution (10% BSA) was added to 2 volumes of the resultant mixed solution and the obtained mixture was stirred for about 1 hour.
   3) After dialysis against 5 mM MOPS (pH 7.0) solution, the dialyzed mixture was diluted to 3 Abs/ml (600 nm) to obtain a # 16 antibody-latex complex (# 16Lx)-containing solution.

### [Experimental Example 1]

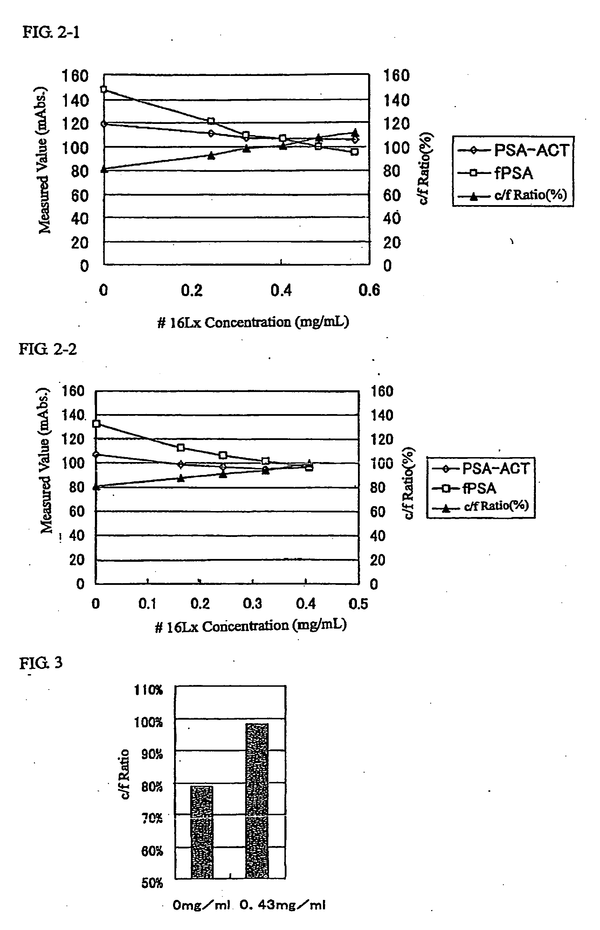

Adjustment of the c/f ratio by addition of # 16 antibody-latex complex (# 16Lx) (1-1)
Agglutination reactions were performed by use of fPSA or PSA-ACT of the same molar concentration, which corresponds to 40 ng/ml of fPSA as an assaying sample and also by use of both # 14Lx and # 17Lx as a second reagent to the basic formula of the following PSA assaying reagent, concentrations of fPSA and PSA-ACT, respectively, were measured, and a concentration ratio PSA-ACT/fPSA (c/f ratio) being about 80% was obtained. Here, since variation of the mixing ratio of # 14Lx and # 17Lx led to substantially no change in the c/f ratio, # 14Lx-containing solution: # 17Lx-containing solution = 2 : 1 was selected and to this was added # 16Lx to a concentration of 0, 0.24, 0.32, 0.41, 0.49, or 0.57 mg/ml to measure c/f ratio at each addition amount of # 16Lx. The results obtained are shown in Fig. 2-1.
Fig. 2-1 indicates that with increasing addition amount of # 16Lx, the c/f ratio increases from about 80% to about 110%. When the condition under which the c/f ratio was 100%, that is, the condition under which fPSA and PSA-ACT exhibit equimolarity was selected, the mixing ratio of carriers was calculated to be # 14Lx-containing solution: # 17Lx-containing solution:# 16Lx-containing solution = 2 : 1 : about 0.27.

### (1) Basic formula of PSA assaying reagent

First reagent (buffer)
   0.5M KCl
   0.1% BSA (proliant)
   30 mM HEPES buffer pH 7.0
Second regent (antibody sensitized latex solution)
   anti-PSA mouse monoclonal antibody sensitized latex particle(*),
   5 mM MOPS pH 7.0
   * mixing ratio of three kinds of antibody sensitized latex particle
   # 14 Lx-containing solution : # 17 Lx-containing solution : # 16 Lx-containing
   solution = 2 : 1 : about 0.27

### (2) Measurement condition

Measurement device: H7170
Parameter:
   Analysis two-point end (measurement point 19 to 34)
   Fluid volume 8/100/100
   Measurement wavelength 570(main)/800(sub)
   Calibration spline

### (3) Assaying sample

fPSA and PSA-ACT (both manufactured by Fitzgerald Co.) were dissolved in 1% BSA, 0.1% NaN₃/PBS to prepare a sample.

### [Experimental Example 1-2]

Adjustment of the c/f ratio by addition of # 16 antibody-latex complex (# 16Lx) (1-2) In relation to the adjustment of the c/f ratio by addition of the # 16 Atibody-latex complex # 16Lx, experiments were carried out by increasing the levels of # 16Lx in the cases where # 16Lx addition amounts were low.
The c/f ratio was assayed at each addition amount of # 16Lx in the same manner as in Experimental Example 1 except that level of the concentration of assaying sample in Experimental Example 1 (40 ng/ml) was changed to 36 ng/ml and levels of addition amounts of # 16Lx were changed to 0, 0.16, 0.24, 0.32, or 0.40 mg/ml. The results obtained are shown in Fig. 2-2.
Figs. 2-1 and 2-2 indicate that appropriate adjustment of the addition amount of # 16Lx enables the c/f ratio to be adjusted between 90% and 110%.

### [Experimental Example 2]

### Storage stability

Under the conditions under which the c/f ratio obtained in Experimental Example 1 was 100%, the reagents were stored at 4°C and the time course of the c/f ratio was measured. As a result, the c/f ratio was maintained to be 90% to 110% during storage time over 7 months.

### [Experimental Example 3]

### Effect of addition of free antibody # 16

The c/f ratios were measured in the same manner as in Experimental Example 1 except that the antibody #16 was not sensitized to the carrier and left to be in a free state. The results obtained are shown in Fig. 3. Using the basic formula of the PSA assaying reagent described in Experimental Example 1, # 16 (0.43 mg/ml) as a second reagent as well as # 14Lx and # 17Lx were added.
Using fPSA or PSA-ACT at the same molar concentration, which corresponds to 8.7 ng/ml of PSA as a sample, signal ratio of fPSA and PSA-ACT, or c/f ratio, were obtained. As a result, it revealed that free antibody # 16 not sensitized to a carrier also had an effect of improving the c/f ratio to near 100%.

### [Experimental Example 4]

### Combination with another monoclonal antibody

The c/f ratio was obtained in the same manner as in Experimental Example 1 except that in stead of the combination of monoclonal antibodies # 14 and # 17, a combination of monoclonal antibodies # 17 and # 36 was used. As samples, fPSA or PSA-ACT of the same molar concentration, which corresponds to 30ng/ml of fPSA, are used. The antibody #16 (0.43 mg/ml) was added to the mixture of monoclonal antibodies #17 and #36. As a result, it was confirmed that when the combination of antibodies in the present Experimental Example was used, the c/f ratio was also increased.

### [Experimental Example 5]

### Influence of latex particle size on c/f ratio

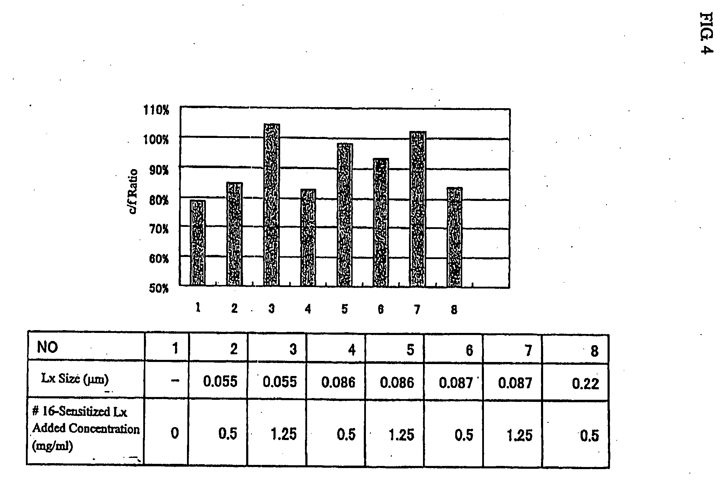

Latexes having a size of 0.055, 0.086, 0.087, or 0.22 µm and sensitized with the monoclonal antibody # 16 were added in a concentration of 0, 0.5 mg/ml, or 1.25 mg/ml and the c/f ratios were each assayed in the same manner as in Experimental Example 1. The results obtained are shown in Fig. 4.
The results shown in Fig. 4 indicate that the c/f ratio of the antibody sensitized to latexes of a particle size of 0.055 to 0.22 µm had an effect of improving the c/f ratio to 100% in a concentration-dependent manner.

### Industrial Applicability

The reagent of assaying PSA according to the present invention can be obtained by appropriately combining antibodies obtained by a conventional method without using special antibodies. Therefore, use of the assaying reagent of the present invention enables simple and accurate assay of PSA. Further, since the reagent of assaying antigen of the present invention can be applied to a general-purpose automatic analyzer, the present invention can greatly contribute to reduce economical, temporal and physical loads to patients upon tests.

## Claims

1. A reagent for assaying PSA by an immunological agglutination reaction, comprising:
at least three types of antibodies capable of reacting with both prostate specific antigen-α1-antichymotrypsin complex (PSA-ACT) and free prostate specific antigen (fPSA) and having different recognition sites therefor, wherein:
at least two types of antibodies out of the at least three types of antibodies are each sensitized to a carrier and react with both PSA-ACT and fPSA; and
mixing ratios of the at least three antibodies are adjusted so that both PSA-ACT and fPSA can be determined with a predetermined molar reactivity when the antibodies each react with PSA-ACT and fPSA.

2. The reagent for assaying PSA according to claim 1, wherein the predetermined molar reactivity is equimolarity (equimolar reactivity).

3. The reagent for assaying PSA according to claim 1 or claim 2, wherein the reagent includes three types of antibodies.

4. The reagent for assaying PSA according to any one of claims 1 to 3, wherein all of the antibodies are sensitized to respective carriers.

5. The reagent for assaying PSA according to any one of claims 1 to 4, wherein the carrier sensitized with one of the antibodies and the carrier sensitized with another of the antibodies have different particle sizes from each other.

6. The reagent for assaying PSA according to any one of claims 1 to 5, wherein the antibodies are monoclonal antibodies.

7. A reagent for assaying PSA by an immunological agglutination reaction, comprising:
latexes sensitized with three types of monoclonal antibodies capable of reacting with both PSA-ACT and fPSA and having different recognition sites therefor, wherein:
latexes sensitized with two types of antibodies out of the three types of antibodies have a particle size larger than that of the latex sensitized with the third antibody; and
mixing ratios of the antibodies are adjusted so that both PSA-ACT and fPSA can be determined with a predetermined molar reactivity when the antibodies each react with PSA-ACT and fPSA

8. The reagent for assaying PSA according to claim 7, wherein the carriers sensitized with the respective antibodies are included in a single reagent.

9. The reagent for assaying PSA according to claim 7 or claim 8, wherein the latex sensitized with the third antibody has a particle size of 0.02 µm to 0.22 µm.

10. A method of producing a PSA assaying reagent by an immunological agglutination reaction, comprising:
providing three carriers sensitized with respective antibodies capable of reacting with both PSA-ACT and fPSA and having different recognition sites therefor;
setting in advance a mixing ratio of two different antibody-sensitized carriers out of the three types of antibody-sensitized carriers to a predetermined range; and
then, adding the third antibody-sensitized carrier to a mixture of the two types of antibody-sensitized carriers thereby performing adjustment so that both PSA-ACT and fPSA can be determined with a predetermined molar reactivity when the antibodies each react with PSA-ACT and fPSA

11. The method according to claim 10, wherein the predetermined molar reactivities are equimolarities.

12. A method of assaying PSA in an analyte by an immunological agglutination reaction, comprising:
assaying PSA in an analyte using a reagent for assaying PSA, wherein:
the reagent for assaying PSA includes carriers sensitized with three types of antibodies capable of reacting with both PSA-ACT and fPSA having different recognition sites therefor;
carriers sensitized with two types of antibodies out of the three types of antibodies have a larger particle size than that of a carrier sensitized with the third antibody; and
a mixing ratio of each of the antibodies is adjusted so that both PSA-ACT and fPSA can be determined with a predetermined molar reactivity when the antibodies each react with PSA-ACT and fPSA

13. The method of assaying PSA in an analyte according to claim 12, wherein the predetermined molar reactivities are equimolarities.

## Patentansprüche

1. Reagenz zur Bestimmung von PSA mittels einer immunologischen Agglutinationsreaktion, umfassend:
mindestens drei Arten von Antikörpern, die in der Lage sind, sowohl mit prostataspezifischem Antigen-α1-Antichymotrypsin-Komplex (PSA-ACT) als auch freiem prostataspezifischen Antigen (fPSA) zu reagieren, und unterschiedliche Erkennungsstellen dafür aufweisen, wobei:
mindestens zwei Arten von Antikörpern von den mindestens drei Arten von Antikörpern jeweils auf einen Träger sensibilisiert sind und sowohl mit PSA-ACT als auch fPSA reagieren; und
die Mischungsverhältnisse der mindestens drei Antikörper angepasst sind, sodass sowohl PSA-ACT als auch fPSA mit einer vorbestimmten molaren Aktivität (molar reactivity) bestimmt werden kann, wenn die Antikörper jeweils mit PSA-ACT und fPSA ragieren.

2. Das Reagenz zur Bestimmung von PSA nach Anspruch 1, wobei die vorbestimmte molare Aktivität Äquimolarität (äquimolare Aktivität (equimolar reactivity)) ist.

3. Das Reagenz zur Bestimmung von PSA nach Anspruch 1 oder Anspruch 2, wobei das Reagenz drei Arten von Antikörpern umfasst.

4. Das Reagenz zur Bestimmung von PSA nach einem der Ansprüche 1 bis 3, wobei alle Antikörper auf die jeweiligen Träger sensibilisiert sind.

5. Das Reagenz zur Bestimmung von PSA nach einem der Ansprüche 1 bis 4, wobei der Träger, der mit einem der Antikörper sensibilisiert ist, und der Träger, der mit einem anderen der Antikörper sensibilisiert ist, Teilchengrößen haben, die unterschiedlich voneinander sind.

6. Das Reagenz zur Bestimmung von PSA nach einem der Ansprüche 1 bis 5, wobei die Antikörper monoklonale Antikörper sind.

7. Reagenz zur Bestimmung von PSA mittels einer immunologischen Agglutinationsreaktion, umfassend:
Latizes, die mit drei Arten von monoklonalen Antikörpern, welche in der Lage sind, sowohl mit PSA-ACT als auch fPSA zu reagieren, und unterschiedliche Erkennungsstellen dafür aufweisen, sensibilisiert sind, wobei:
Latizes, die sensibilisiert sind mit zwei Arten von Antikörpern aus den drei Arten von Antikörpern, eine größere Teilchengröße haben als der Latex, welcher mit dem dritten Antikörper sensibilisiert ist; und
die Mischungsverhältnisse der Antikörper angepasst sind, sodass sowohl PSA-ACT als auch fPSA mit einer vorbestimmten molaren Aktivität (molar reactivity) bestimmt werden kann, wenn die Antikörper jeweils mit PSA-ACT und fPSA ragieren.

8. Das Reagenz zur Bestimmung von PSA nach Anspruch 7, wobei die Träger, welche mit den entsprechenden Antikörpern sensibilisiert sind, in einem einzigen Reagenz enthalten sind.

9. Das Reagenz zur Bestimmung von PSA nach Anspruch 7 oder Anspruch 8, wobei der Latex, welcher mit dem dritten Antikörper sensibilisiert ist, eine Teilchengröße von 0,02 µm bis 0,22 µm hat.

10. Verfahren zur Herstellung eines Reagenz zur Bestimmung von PSA mittels immunologischer Agglutinationsreaktion, umfassend:
das Bereitstellen von drei Trägern, die mit entsprechenden Antikörpern, welche in der Lage sind, sowohl mit PSA-ACT als auch fPSA zu reagieren, und unterschiedliche Erkennungsstellen dafür aufweisen, sensibilisiert sind;
das vorhergehende Einstellen eines Mischungsverhältnisses von zwei verschiedenen Antikörper-sensibilisierten Trägern aus den drei Arten von Antikörper-sensibilisierten Trägern auf einen vorbestimmten Bereich; und
dann, das Zugeben des dritten Antikörper-sensibilisierten Trägers zu einer Mischung der zwei Arten von Antikörper-sensibilisierten Trägern, wodurch eine Anpassung erfolgt, sodass sowohl PSA-ACT als auch fPSA mit einer vorbestimmten molaren Aktivität (molar reactivity) bestimmt werden kann, wenn die Antikörper jeweils mit PSA-ACT und fPSA reagieren.

11. Das Verfahren nach Anspruch 10, wobei die vorbestimmten molaren Aktivitäten Äquimolaritäten sind.

12. Verfahren zur Bestimmung von PSA in einem Analyt mittels einer immunologischen Agglutinationsreaktion, umfassend:
das Bestimmen von PSA in einem Analyt unter Verwendung von einem Reagenz zur Bestimmung von PSA, wobei:
das Reagenz zur Bestimmung von PSA Träger enthält, die mit drei Arten von Antikörpern, welche in der Lage sind, sowohl mit PSA-ACT als auch fPSA zu reagieren, [und] unterschiedliche Erkennungsstellen dafür aufweisen, sensibilisiert sind;
die Träger, die mit zwei Arten von Antikörpern aus den drei Arten von Antikörpern sensibilisiert sind, eine größere Teilchengröße haben als ein Träger, der mit dem dritten Antikörper sensibilisiert ist;und
ein Mischungsverhältnis von jedem der Antikörper angepasst ist, sodass sowohl PSA-ACT als auch fPSA mit einer vorbestimmten molaren Aktivität (molar reactivity) bestimmt werden kann, wenn die Antikörper jeweils mit PSA-ACT und fPSA reagieren.

13. Das Verfahren zur Bestimmung von PSA in einem Analyt nach Anspruch 12, wobei die vorbestimmten molaren Aktivitäten Äquimolaritäten sind.

## Revendications

1. Réactif de dosage de PSA par une réaction d'agglutination immunologique, comprenant :
au moins trois types d'anticorps capables de réagir à la fois avec un complexe antigène spécifique de la prostate-α1-antichymotrypsine (PSA-ACT) et un antigène spécifique de la prostate libre (fPSA) et ayant des sites de reconnaissance différents à cet effet, dans lequel :
au moins deux types d'anticorps parmi les au moins trois types d'anticorps sont chacun sensibilisés à un support et réagissent à la fois avec le PSA-ACT et le fPSA ; et
des rapports de mélange des au moins trois anticorps sont réglés de sorte que les deux PSA-ACT et fPSA puissent être déterminés avec une réactivité molaire prédéterminée lorsque chaque anticorps réagit avec le PSA-ACT et le fPSA.

2. Réactif de dosage de PSA selon la revendication 1, dans lequel la réactivité molaire prédéterminée est une équimolarité (réactivité équimolaire).

3. Réactif de dosage de PSA selon la revendication 1 ou 2, dans lequel le réactif comporte trois types d'anticorps.

4. Réactif de dosage de PSA selon l'une quelconque des revendications 1 à 3, dans lequel tous les anticorps sont sensibilisés à des supports respectifs.

5. Réactif de dosage de PSA selon l'une quelconque des revendications 1 à 4, dans lequel le support sensibilisé avec l'un des anticorps et le support sensibilisé avec un autre des anticorps présentent des dimensions de particules différentes l'une de l'autre.

6. Réactif de dosage de PSA selon l'une quelconque des revendications 1 à 5, dans lequel les anticorps sont des anticorps monoclonaux.

7. Réactif de dosage de PSA par une réaction d'agglutination immunologique, comprenant :
des latex sensibilisés avec trois types d'anticorps monoclonaux capables de réagir à la fois avec le PSA-ACT et le fPSA et ayant des sites de reconnaissance différents à cet effet, dans lequel :
des latex sensibilisés avec deux types d'anticorps parmi les trois types d'anticorps présentent une dimension de particules supérieure à celle des latex sensibilisés avec le troisième anticorps ; et
des rapports de mélange des anticorps sont réglés de sorte que les deux PSA-ACT et fPSA puissent être déterminés avec une réactivité molaire prédéterminée lorsque chaque anticorps réagit avec le PSA-ACT et le fPSA.

8. Réactif de dosage de PSA selon la revendication 7, dans lequel les supports sensibilisés avec les anticorps respectifs sont inclus dans un seul réactif.

9. Réactif de dosage de PSA selon la revendication 7 ou 8, dans lequel le latex sensibilisé avec le troisième anticorps présente une dimension de particules de 0,02 µm à 0,22 µm.

10. Procédé de production d'un réactif de dosage de PSA par une réaction d'agglutination immunologique, comprenant le fait :
de fournir trois supports sensibilisés avec des anticorps respectifs capables de réagir à la fois avec le PSA-ACT et le fPSA et ayant des sites de reconnaissance différents à cet effet ;
de régler à l'avance un rapport de mélange de deux différents supports sensibilisés avec les anticorps parmi les trois types de supports sensibilisés avec les anticorps à une plage prédéterminée ; et
ensuite, d'ajouter le troisième support sensibilisé avec les anticorps à un mélange de deux types de supports sensibilisés avec les anticorps effectuant ainsi un réglage de sorte que les deux PSA-ACT et fPSA puissent être déterminés avec une réactivité molaire prédéterminée lorsque chaque anticorps réagit avec le PSA-ACT et le fPSA.

11. Procédé selon la revendication 10, dans lequel les réactivités molaires prédéterminées sont des équimolarités.

12. Procédé de dosage de PSA dans un analyte par une réaction d'agglutination immunologique, comprenant le fait :
de doser un PSA dans un analyte en utilisant un réactif de dosage de PSA, dans lequel :
le réactif de dosage de PSA comporte des supports sensibilisés avec trois types d'anticorps capables de réagir à la fois avec le PSA-ACT et le fPSA ayant des sites de reconnaissance différents à cet effet ;
des supports sensibilisés avec deux types d'anticorps parmi les trois types d'anticorps présentent une dimension de particules supérieure à celle d'un support sensibilisé avec le troisième anticorps ; et
un rapport de mélange de chacun des anticorps est réglé de sorte que les deux PSA-ACT et fPSA puissent être déterminés avec une réactivité molaire prédéterminée lorsque chaque anticorps réagit avec le PSA-ACT et le fPSA.

13. Procédé de dosage de PSA dans un analyte selon la revendication 12, dans lequel les réactivités molaires prédéterminées sont des équimolarités.
